# FASCICULE DE BREVET EUROPEEN

(11) **EP 2 858 584 B1**
(45) Date de publication et mention de la délivrance du brevet: **30.03.2016**
(21) Numéro de dépôt: 13729997.0
(22) Date de dépôt: 28.05.2013
(51) Int. Cl.: A61B 17/70

(54) **IMPLANT ILIO-SACRE DE CONNEXION A UN SYSTEME D'OSTEOSYNTHESE RACHIDIENNE**
ILIOSAKRALIMPLANTAT ZUR VERBINDUNG MIT EINEM SPINALEN OSTEOSYNTHESESYSTEM
ILIOSACRAL IMPLANT FOR CONNECTION TO A SPINAL OSTEOSYNTHESIS SYSTEM

(30) Priorité: 07.06.2012 FR 1201645
(43) Date de publication de la demande: 15.04.2015
(73) Titulaire: Euros, 13600 La Ciotat (FR)
(72) Inventeur: MILADI, Lofti, F-92340 Bourg La Reine (FR)
(74) Mandataire: Orsini, Fabienne
(86) Numéro de dépôt international: PCT/FR2013/051190
(87) Numéro de publication internationale: WO 2013/182780

(56) Documents cités:
- FR-A1- 2 794 962
- US-B2- 8 052 726

## Description

### DOMAINE TECHNIQUE AUQUEL SE RAPPORTE L'INVENTION

La présente invention concerne de manière générale les systèmes d'ostéosynthèse rachidienne (ou « de connexion spinale ») permettant d'immobiliser au moins une vertèbre relativement au bassin d'un patient.

Elle concerne plus particulièrement un implant ilio-sacré pour système d'ostéosynthèse rachidienne, qui comprend un élément de connexion comportant :
- un collier qui présente un trou axial traversant qui est destiné à être traversé par un élément d'ancrage, et un trou transversal traversant qui débouche dans le trou axial,
- un réceptacle qui est solidarisé au collier et qui présente un logement d'accueil d'une tige de liaison du système d'ostéosynthèse rachidienne, et
- un arrêtoir qui est destiné à bloquer l'élément d'ancrage dans le trou axial du collier.

### ARRIERE-PLAN TECHNOLOGIQUE

Pour corriger des déviations importantes de la colonne vertébrale telles que les scolioses ou les cyphoses, il était connu d'utiliser des tiges de liaison rigides permettant d'immobiliser toutes les vertèbres de la colonne vertébrale dans une position prédéterminée. Pour ce faire, les tiges de liaison étaient cintrées suivant une courbure adéquate, puis étaient fixées parallèlement à la colonne vertébrale au moyen d'une pluralité d'implants vissés ou accrochés à l'ensemble des vertèbres de la colonne vertébrale.

Plus récemment, une nouvelle technique moins invasive et plus légère pour le patient a été mise au point. Cette nouvelle technique consiste à utiliser un nombre réduit d'implants, dont une partie d'entre eux est installée sur une ou plusieurs des vertèbres thoraciques du patient, et dont l'autre partie est vissée dans l'os iliaque du bassin du patient.

On parle alors de « montage suspendu » en ce sens que les vertèbres thoraciques sont suspendues aux extrémités hautes des tiges de liaison fixées par leurs extrémités basses au bassin du patient.

Pour fixer chaque tige de liaison au bassin du patient, il est alors connu du document US 8 052 726 d'utiliser un implant ilio-sacré du type cité en introduction, dont l'élément d'ancrage est une vis vissée dans le bassin du patient.

Selon ce document, l'élément de connexion de l'implant est formé d'une seule pièce. Le collier, qui est enfilé sur la vis, et le réceptacle, qui reçoit la tige de liaison, sont alors monoblocs.

L'arrêtoir, qui permet de bloquer la vis dans le collier, est quant à lui formé par une bague fendue engagée dans une gorge prévue à l'intérieur du trou axial du collier.

Il est en outre prévu une fenêtre dans le fond du réceptacle, par laquelle la face externe de la bague fendue débouche dans le logement d'accueil. Ainsi, lorsque la tige de liaison est rapportée dans son logement d'accueil, elle vient s'appuyer contre la face externe de cette bague fendue, de manière que la bague se resserre autour de la vis et la bloque dans le trou axial.

L'inconvénient majeur de cet implant est qu'il arrive que la bague sorte de sa gorge, soit lors de la mise en place de l'implant sur le bassin du patient, ce qui nécessite le remplacement de l'implant, soit plus tard au cours de la vie du patient, ce qui nécessite une nouvelle opération chirurgicale pour extraire la bague et l'implant et les remplacer.

Un autre inconvénient de cet implant est que, au cours de l'opération chirurgicale, l'élément de connexion reste mobile par rapport à la vis jusqu'à ce que la tige de liaison soit rapportée et fixée dans le réceptacle, ce qui risque d'entraîner un dévissage de la vis avant la mise en place de cette tige de liaison.

Un autre inconvénient est engendré par le fait que le système de blocage par la bague n'est complètement fiable de sorte que dans certains cas, la vis peut se dévisser spontanément avec le temps et nécessiter une nouvelle intervention pour son changement.

### OBJET DE L'INVENTION

Afin de remédier aux inconvénients précités de l'état de la technique, la présente invention propose un nouvel implant ilio-sacré, dans lequel l'arrêtoir est parfaitement solidarisé à l'élément de connexion et peut être actionné avant la mise en place de la tige de liaison.

Plus particulièrement, on propose selon l'invention un implant tel que défini dans l'introduction, dans lequel l'arrêtoir comporte un corps qui est monté mobile au travers du trou transversal du collier, et une partie de manoeuvre qui est accessible par le logement d'accueil du réceptacle et qui permet de déplacer le corps depuis une première position dans laquelle il laisse l'élément d'ancrage libre de se mouvoir au travers du trou axial du collier jusqu'à une seconde position dans laquelle il bloque l'élément d'ancrage dans le trou axial du collier.

Grâce à la position de son corps dans le trou transversal du collier, l'arrêtoir ne peut donc pas se désolidariser de l'élément de connexion, de sorte que l'implant ne peut se désassembler ni pendant l'opération, ni après.

Par ailleurs, grâce à l'accessibilité de sa partie de manoeuvre, l'arrêtoir peut être manoeuvré indépendamment de la tige de liaison, ce qui permet de bloquer le réceptacle de l'élément de connexion par rapport à l'élément d'ancrage avant la mise en place de la tige de liaison.

Préférentiellement, le réceptacle est solidarisé au collier au moyen d'une liaison rotule.

En effet, un autre inconvénient de l'état de la technique est dû au fait que la partie de connexion étant monobloc, il était nécessaire de cintrer la tige pour permettre son introduction dans le réceptacle dans les cas fréquents où il y a un décalage entre les implants.

Ici, cette liaison confère une plus grande liberté pour la mise en place de la tige de liaison dans le réceptacle, ce qui facilite l'opération.

D'autres caractéristiques avantageuses et non limitatives de l'implant conforme à l'invention sont les suivantes :
- le trou transversal du collier est au moins en partie taraudé et ledit corps comporte une tige filetée vissée dans le trou transversal du collier ;
- la partie de manoeuvre comporte une empreinte en creux, dans laquelle un outil de vissage est adapté à être engagé pour visser la tige filetée dans le trou transversal du collier ;
- le logement d'accueil du réceptacle est délimité du côté du collier par un fond qui présente une ouverture dans laquelle est engagé l'arrêtoir ;
- l'arrêtoir comporte une tête au moins partiellement sphérique ou conique, formant la partie mâle de ladite liaison rotule ;
- le fond du réceptacle présente, autour de ladite ouverture, une face intérieure sphérique ou conique sur laquelle s'appuie la tête de l'arrêtoir et qui forme la partie femelle de ladite liaison rotule ;
- l'élément de connexion comporte un moyen de retenue monté mobile dans le logement d'accueil du réceptacle, entre une position de libération dans laquelle la liaison rotule est libre et une position de retenue dans laquelle la liaison rotule est bloquée pour rendre le collier et le réceptacle fixes l'un par rapport à l'autre ;
- le logement d'accueil du réceptacle débouchant vers l'extérieur par deux ouvertures latérales en forme de berceaux et situées en regard l'une de l'autre, ledit élément de retenue comporte une bague dont une face s'appuie, en position de retenue, sur la tête de l'arrêtoir, et dont une partie s'étend, en position de libération, au-dessus des fonds des berceaux formés par lesdites deux ouvertures latérales ;
- ladite bague est fendue et est engagée dans une gorge périphérique située en creux dans le réceptacle, à l'intérieur du logement d'accueil ;
- ledit logement d'accueil débouchant vers l'extérieur à l'opposé du fond par une ouverture d'introduction taraudée, il est prévu un verrou qui est vissé dans ladite ouverture d'introduction pour immobiliser ladite tige de liaison contre la bague et contre les fonds des berceaux formés par lesdites deux ouvertures latérales ;
- il est prévu un élément d'ancrage comportant une tige allongée qui est adaptée à se fixer à l'os iliaque d'un patient et qui est engagée dans le trou axial du collier.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue en perspective du bassin d'un patient et de certaines de ses vertèbres lombaires, dont l'une d'entre elles est bloquée par rapport au bassin à l'aide d'une tige de liaison, d'un implant rachidien et d'un implant ilio-sacré selon l'invention ;
- la figure 2 est une vue éclatée de l'élément de connexion de l'implant ilio-sacré de la figure 1 ;
- la figure 3 est une vue assemblée de l'élément de connexion de la figure 2 ;
- la figure 4 est une vue éclatée de l'implant ilio-sacré de la figure 1, sur laquelle apparaît également la tige de liaison de la figure 1 ;
- la figure 5 est une vue en coupe axiale de l'implant ilio-sacré de la figure 1, sur laquelle la liaison rotule de l'élément de connexion est laissée libre et sur laquelle l'élément d'ancrage est laissé mobile par rapport à l'élément de connexion ;

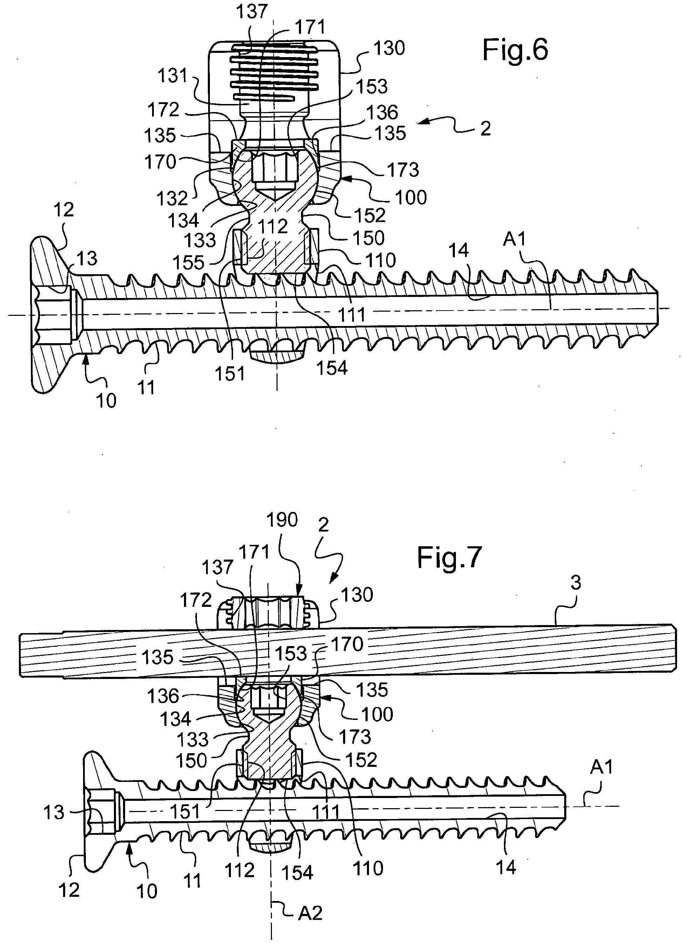

- la figure 6 est une vue en coupe axiale de l'implant ilio-sacré de la figure 1, sur laquelle la liaison rotule de l'élément de connexion est laissée libre et sur laquelle l'élément d'ancrage est bloqué par rapport à l'élément de connexion ;
- la figure 7 est une vue en coupe axiale de l'implant ilio-sacré de la figure 1, sur laquelle la liaison rotule de l'élément de connexion est bloquée et sur laquelle l'élément d'ancrage est bloqué par rapport à l'élément de connexion.

La description qui va suivre porte sur un système de connexion 1 permettant à un chirurgien de bloquer une ou plusieurs vertèbres d'une colonne vertébrale d'un patient par rapport au bassin de ce patient. Elle permet notamment de redresser la colonne vertébrale du patient lorsque celle-ci présente une scoliose ou une cyphose prononcée.

Elle permet en particulier de bloquer les vertèbres cervicales du patient par rapport à son bassin, au moyen d'une ou de deux tiges de liaison rigides, préalablement cintrées de manière adéquate.

Elle permet plus généralement de bloquer toute vertèbre du patient par rapport à son bassin, et de corriger la bascule de ce dernier dans les trois plans de l'espace.

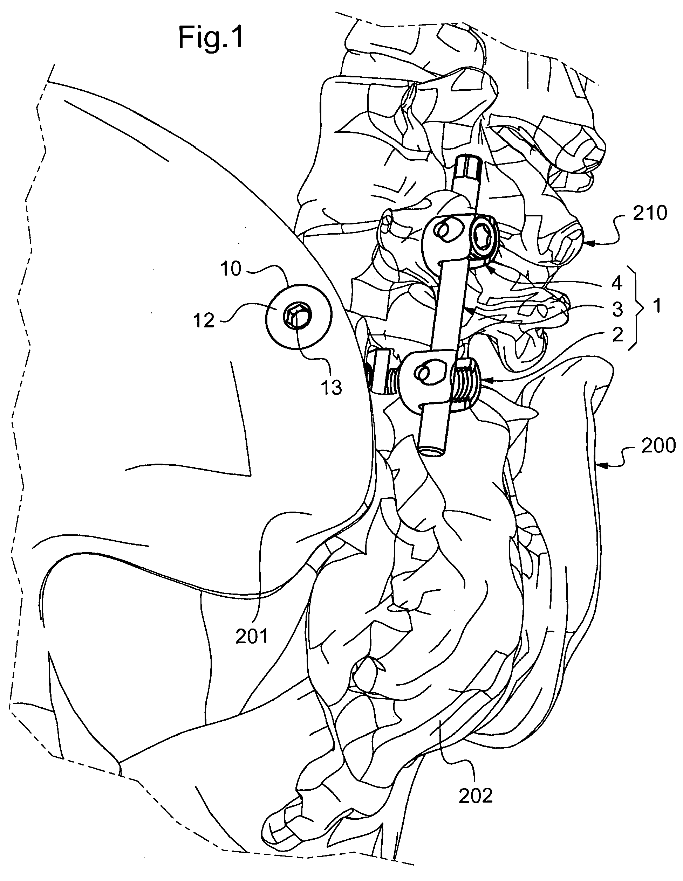

Sur la figure 1, pour la clarté des figures, on a représenté un montage permettant de bloquer la dernière des vertèbres lombaires 210 du patient (la vertèbre L5) par rapport à l'os iliaque 201 gauche et au sacrum 202 du patient.

Le système de connexion 1 comporte à cet effet ici une tige de liaison 3 connectée, d'une part, à la vertèbre lombaire 210 au moyen d'un implant rachidien 4, et, d'autre part, à l'os iliaque 201 et au sacrum 202 du patient au moyen d'un implant ilio-sacré 2.

La tige de liaison 3 est une tige en titane, de diamètre environ égal à 5 millimètres.

L'implant rachidien 4 est quant à lui ici une vis pédiculaire, par exemple du type de celle décrite dans le document US 6 254 602.

Cette tige de liaison 3 et cet implant rachidien 4 ne faisant pas en propre l'objet de la présente invention, ils ne seront pas ici décrits plus en détail.

L'invention porte plus particulièrement sur l'implant ilio-sacré 2.

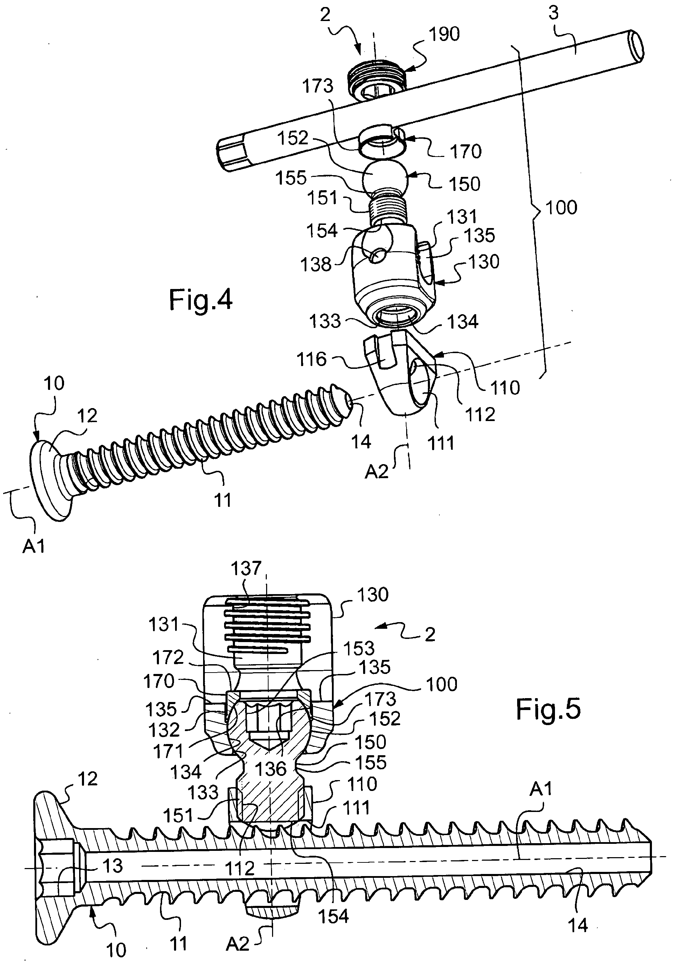

Comme le montre bien la figure 4, cet implant ilio-sacré 2 est ici composé de deux éléments, à savoir un élément d'ancrage 10 à fixer au bassin du patient, et un élément de connexion 100 faisant office de connecteur entre l'élément d'ancrage 10 et la tige de liaison 3.

L'élément d'ancrage, tel qu'il apparaît sur les figures 4 et 5, se présente sous la forme d'une vis 10, avec une tête 12 et une tige filetée 11 allongée suivant un axe longitudinal A1. Cette vis présente ici un diamètre extérieur d'environ 7 millimètres.

Le profil du filetage de la tige filetée 11 est conçu de telle sorte que la vis 10 est auto-taraudeuse et qu'elle peut parfaitement pénétrer et s'ancrer dans l'os iliaque et dans le sacrum du patient.

La tête 12 de la vis 10 présente une première face, celle raccordée à la tige filetée 11, qui est légèrement incurvée vers la tige filetée 11, et une seconde face, opposée à la première, qui est sensiblement plane pour réduire au mieux l'irritation des tissus et de la peau du patient.

La seconde face présente en son centre une empreinte 13 en creux, permettant de recevoir l'extrémité d'un outil de vissage. Ici, cette empreinte 13 est hexagonale, de manière à pouvoir recevoir l'extrémité d'une clef allen.

Comme le montre bien la figure 5, la vis 10 est traversée par un canal 14 cylindrique de révolution autour de l'axe longitudinal A1. Comme cela sera exposé dans la suite de cet exposé, ce canal 14 permet de faciliter la mise en place de la vis 10.

Tel qu'il apparaît sur la figure 4, l'élément de connexion 100, qui fait la liaison entre la vis 10 et la tige de liaison 3, comporte quant à lui :
- un collier 110 qui présente un trou axial 111 par lequel il est enfilé sur la tige filetée 11 de la vis 10, et un trou transversal 112 qui débouche latéralement dans le trou axial 111,
- un réceptacle 130 qui est connecté par son fond 131 au collier 110, et qui comprend un logement d'accueil 131 dans lequel est engagée transversalement la tige de liaison 3,
- un verrou 190 qui permet de bloquer la tige de liaison 3 dans le réceptacle 130, et
- un arrêtoir 150 qui permet de bloquer la vis 10 dans le collier 110.

Selon une caractéristique particulièrement avantageuse de l'invention, cet arrêtoir 150 comporte :
- un corps 151 qui est monté mobile au travers du trou transversal 112 pour déboucher par une première de ses extrémités dans le logement axial 111, et
- une partie de manoeuvre 153 qui est accessible par le logement d'accueil 131 du réceptacle 130 et qui permet de déplacer le corps 151 depuis une première position (fixe ou non) dans laquelle la vis 10 est libre de se déplacer par rapport au collier 110 jusqu'à une seconde position stable et fixe dans laquelle la vis 10 est bloquée dans le collier 110.

Selon une autre caractéristique avantageuse de cette invention, le réceptacle 130 est solidarisé au collier 110 au moyen d'une liaison rotule.

Préférentiellement alors, l'arrêtoir 150 comporte, à la seconde extrémité 155 du corps 151, une tête 152 au moins partiellement sphérique ou conique, qui forme la partie mâle de cette liaison rotule et qui est à cet effet engagée dans le logement d'accueil 131 du réceptacle 130 au travers d'une ouverture d'accès 133 pratiquée dans le fond 132 de ce réceptacle 130.

La partie femelle de la liaison rotule est alors formée, d'un côté, par la face interne du fond 132 du réceptacle 130 (autour de l'ouverture d'accès 133), et, de l'autre, par une bague 170 engagée dans le logement d'accueil 131 du réceptacle 130.

Les différents éléments de l'implant ilio-sacré 2, tels qu'ils sont représentés sur les différentes figures, vont maintenant être décrits plus en détail.

Dans la suite de la description, les termes « avant » et « arrière » seront utilisés par rapport à la direction du regard du chirurgien qui pratique l'opération vers le patient. Dans l'exemple représenté sur les figures, l'avant désignera alors le côté tourné vers le patient et l'arrière désignera le côté tourné à l'opposé, vers le chirurgien.

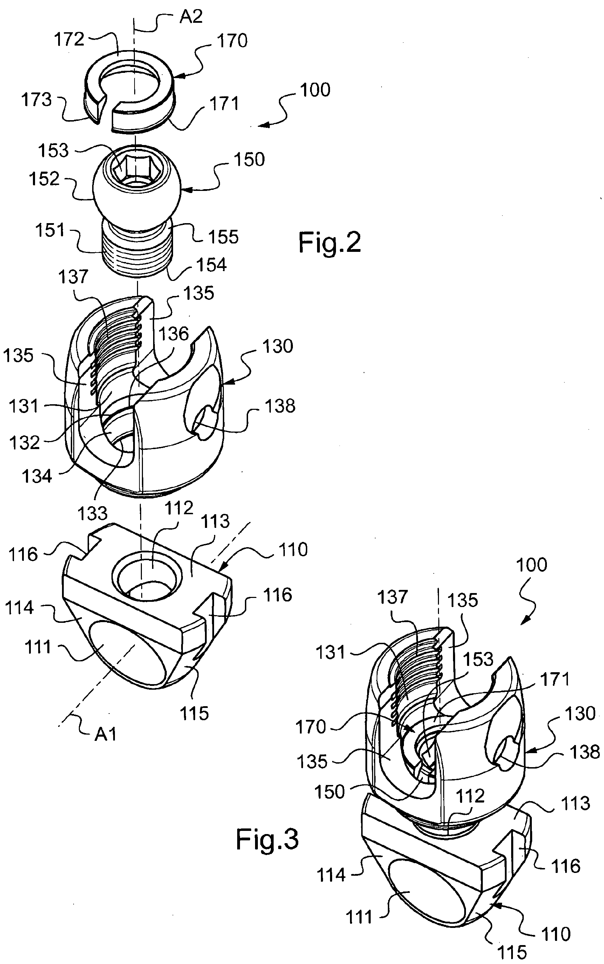

Comme le montre la figure 2, le collier 110 se présente sous la forme d'un monolithe, avec une face arrière 113 plane, deux faces latérales 114 opposées, planes et perpendiculaires à la face arrière 113, et une face avant 115 bombée. Les faces latérales 114 présentent des formes globalement triangulaires, avec une base raccordée à la face arrière 113 et un sommet arrondi. La face avant 115 est alors bombée en forme de U, de manière à border les deux faces latérales 114, depuis un côté jusqu'à l'autre de la face arrière 113.

La face arrière 115 du collier 110 présente ici deux encoches 116 opposées, situées à proximité de la face arrière 113, pour faciliter la mise en place du collier 110 sur le bassin du patient au moyen d'une pince ou d'un outil adapté.

Le trou axial 111 pratiqué dans ce collier 110 s'étend alors entre les deux faces latérales 114 et il présente ici une forme cylindrique de révolution autour d'un axe longitudinal A1 orthogonal à ces deux faces latérales 114.

Ce trou axial 111 présente un diamètre supérieur au diamètre extérieur de la tige filetée 11 de la vis 10, qui est en pratique ici presqu'égal (au jeu près) à celui-ci.

Le trou transversal 112 pratiqué dans ce collier 110 s'étend quant à lui depuis la face arrière 113 jusqu'à l'intérieur du trou axial 111. Il présente ici une forme cylindrique de révolution autour d'un axe transversal A2 qui est perpendiculaire à l'axe longitudinal A1 du trou axial 111 et à la face arrière 113.

Ce trou transversal 112 présente ici un diamètre inférieur au diamètre du trou axial 111, environ égal à 5 millimètres.

Le collier 110 est ici formé d'une seule pièce par moulage, et éventuellement aussi perçage et taraudage, en titane.

Comme le montrent bien les figures 2 et 3, le réceptacle 130 présente une forme allongée, globalement cylindrique autour d'un axe représenté sur les figures comme étant confondu avec l'axe transversal A2.

Ce réceptacle 130 est creux pour délimiter intérieurement le logement d'accueil 131.

Le logement d'accueil 131 présente une forme globalement cylindrique, de diamètre supérieur à celui de la tige de liaison 3, de telle manière que celle-ci puisse être rapportée transversalement dans le logement d'accueil 131.

Le logement d'accueil 131 débouche à l'arrière du réceptacle 130 par une ouverture d'introduction 137. Il comporte par ailleurs, à l'opposé de cette ouverture d'introduction 137, un fond 132 traversé par l'ouverture d'accès 133.

L'ouverture d'accès 133 présente une forme circulaire centrée sur l'axe A2, de diamètre inférieur au diamètre du logement d'accueil 131 et sensiblement égal au diamètre du trou transversal 112 du collier 110.

Le réceptacle 130 présente par ailleurs deux encoches latérales 135, situées en regard et à l'opposé l'une de l'autre par rapport à l'axe A2, qui s'étendent en longueur à partir de l'arrière du réceptacle 130 jusqu'à proximité du fond 132 du réceptacle 130. Les fonds de ces encoches latérales 135 sont arrondis et présentent ainsi des formes de berceaux. Ces deux encoches latérales 135 permettent d'engager la tige de liaison 3 transversalement dans le logement d'accueil 131 du réceptacle 130.

Le réceptacle 130 présente par ailleurs deux empreintes 138 en creux dans sa face externe, situées à l'opposé l'une de l'autre, pour faciliter la manoeuvre du réceptacle 130 au moyen d'une pince ou d'un outil adapté.

Le réceptacle 130 est ici formé d'une seule pièce par moulage, et éventuellement perçage et taraudage, en titane.

Comme cela a été exposé supra, l'arrêtoir 150 présente plusieurs fonctions puisque son corps 151 permet de bloquer la vis 10 dans le collier 110, tandis que sa tête 152 permet de relier le collier 110 au réceptacle 130 via une liaison rotule.

Le corps 151 de l'arrêtoir 150, qui est monté mobile au travers du trou transversal 112 pratiqué dans le collier 110, se présente ici sous la forme d'un axe fileté d'axe A2. En correspondance, le trou transversal 112 du collier 110 est taraudé sur toute sa longueur. Le corps 151 de l'arrêtoir 150 est alors en prise par vissage avec le collier 110.

Ce corps 151 présente une longueur supérieure à la longueur du trou transversal 112 du collier 110, de telle manière que sa première extrémité 154 puisse déboucher à l'intérieur du trou axial 111 du collier 110. De cette manière, lorsque la vis 10 est engagée dans le trou axial 111 du collier 110, il est possible de visser l'arrêtoir 150 dans le trou transversal 112 de manière que la première extrémité 154 de son corps 151 bloque fixement la vis 10 dans le trou axial 111.

La tête 152 de l'arrêtoir 150 se présente quant à elle ici sous la forme d'une sphère centrée sur l'axe A2, de diamètre supérieur à celui du corps 151.

Cette tête 152 est raccordée à la seconde extrémité du corps 151 par un raccord 155, de section réduite par rapport à la section transversale du corps 151.

Lors de l'assemblage de l'élément de connexion 100 de l'implant ilio-sacré 2, l'arrêtoir 150 est engagé par la première extrémité 154 de son corps 151 dans le logement d'accueil 131 du réceptacle 130, au travers de l'ouverture d'introduction 137, de telle manière que son corps 151 traverse entièrement l'ouverture d'accès 133 située dans le fond 132 du réceptacle 130 et que sa tête 152 s'appuie sur la face intérieure 134 du fond 132 du réceptacle 130.

La face intérieure 134 du fond 132 du réceptacle 130 présente à cet effet, autour de l'ouverture d'accès 133, une forme telle que la tête 152 de l'arrêtoir 150 puisse pivoter librement suivant 3 degrés de liberté.

Ici, la face intérieure 134 du fond 132 du réceptacle 130 présente une forme de tronçon de sphère de diamètre égal, au jeu près, au diamètre de la tête 152 de l'arrêtoir 150. En variante, elle pourrait par exemple présenter une forme conique.

Grâce au rétrécissement de section formé par le raccord 155 de l'arrêtoir 150, la tête 152 de l'arrêtoir 150 peut librement pivoter dans le fond 132 du réceptacle 130 sans que le corps 151 de l'arrêtoir 150 ne vienne immédiatement buter contre le bord de l'ouverture d'accès 133 du réceptacle 130.

La partie de manoeuvre de l'arrêtoir 150 qui permet de visser le corps 151 de l'arrêtoir 150 dans le trou transversal 112 du collier 110 est formée par une empreinte 153 en creux dans la tête 152, située à l'opposé du corps 151. Cette empreinte 153 est ainsi prévue pour recevoir l'extrémité d'un outil de vissage engagé au travers du logement d'accueil 131 du réceptacle 130. Ici, cette empreinte 153 est hexagonale, de manière à pouvoir recevoir l'extrémité d'une clef allen.

Ainsi, cette empreinte 153 est située de telle sorte que le chirurgien peut y avoir accès et intervenir sur elle indépendamment de la tige de liaison 3. Le chirurgien peut ainsi bloquer la vis 10 dans le collier 110 lorsqu'il le souhaite, avant de rapporter la tige de liaison 3 dans le logement d'accueil 131 prévu dans le réceptacle 130.

Dans le mode de réalisation représenté, l'arrêtoir 150 est formé d'une seule pièce en titane, par moulage.

La bague 170, qui est prévue à l'arrière de la tête 152 de l'arrêtoir 150, à l'intérieur du logement d'accueil 131 du réceptacle 130, présente plusieurs fonctions. Elle permet tout d'abord de retenir la tête 152 de l'arrêtoir 150 contre le fond 132 du réceptacle 130, si bien qu'elle forme une partie de la liaison rotule. Elle permet également de bloquer cette liaison rotule.

Cette bague 170 est à cet effet montée mobile dans le logement d'accueil 131 du réceptacle 130, entre une position de libération dans laquelle la liaison rotule est laissée libre et une position de retenue dans laquelle la liaison rotule est bloquée pour rendre le collier 110 et le réceptacle 130 fixes l'un par rapport à l'autre.

Ici, la bague 170 présente une forme globalement annulaire autour de l'axe A2. Elle présente une face arrière 172 plane et orthogonale à l'axe A2, des faces latérales externe et interne globalement cylindriques de révolution autour de l'axe A2, et une face arrière globalement conique de révolution autour de l'axe A2 pour s'appliquer contre la tête 152 de l'arrêtoir 150.

Pour son montage mobile dans le logement d'accueil 131 du réceptacle 130, la bague 170 porte sur sa face externe une nervure périphérique 173 de hauteur réduite, qui s'étend le long de son arête avant et qui est engagée dans une gorge périphérique 136 prévue en creux dans la face intérieure du réceptacle 130, à proximité du fond 132.

La bague 170 est ici fendue pour présenter une élasticité nécessaire à son montage dans cette gorge périphérique 136.

La gorge périphérique 136 présente une hauteur légèrement supérieure à la hauteur de la nervure périphérique 173 de la bague 170, ce qui confère à cette dernière la mobilité nécessaire pour libérer ou bloquer la liaison rotule.

La bague 170 est conçue de telle manière que, quelle que soit la position de sa nervure périphérique 173 dans la gorge périphérique 136, sa face supérieure 172 s'étend au-dessus ou à l'affleurement des fonds des berceaux formés par les deux encoches latérales 135 du réceptacle 130. Elle est plus précisément conçue de telle sorte que lorsque sa face avant 171 s'appuie sur la tête de l'arrêtoir 150 et bloque la liaison rotule, sa face arrière 172 s'étend à l'affleurement des fonds des berceaux formés par les deux encoches latérales 135 du réceptacle 130.

De cette manière, lorsque la tige de liaison 3 est rapportée dans le logement d'accueil 131 du réceptacle 130 et s'appuie contre les fonds des berceaux formés par les deux encoches latérales 135 du réceptacle 130, elle appuie également sur la bague 170 et bloque ainsi la liaison rotule.

Dans le mode de réalisation représenté sur les figures, la bague 170 est formée d'une seule pièce par moulage en titane.

Comme le montrent les figures 4 et 5, le verrou 190 est alors prévu pour bloquer la tige de liaison 3 dans le logement d'accueil 131, contre les fonds des berceaux formés par les deux encoches latérales 135 du réceptacle 130.

Ce verrou 190 se présente ici sous le forme d'une vis de verrouillage globalement cylindrique de révolution autour de l'axe A2, filetée sur sa face extérieure pour pouvoir se visser dans un taraudage prévu en correspondance dans l'ouverture d'introduction 137 du réceptacle 130.

Pour visser le verrou 190 dans le réceptacle 130, il est prévu au travers de ce verrou 190 un trou traversant d'axe A2 et de section transversale hexagonale, adapté à recevoir l'extrémité d'une clef allen.

Le verrou 190 est ici formé d'une seule pièce par moulage en titane.

La mise en place de l'implant ilio-sacré 2 sur le bassin du patient est réalisée par le chirurgien de la manière suivante.

Après avoir dégagé les arcs postérieurs du rachis lombosacré, le chirurgien creuse une petite cavité sous la corticale postérieure du sacrum 202, dans l'espace situé entre le pied de l'articulaire L5S1 et le premier trou sacré. Cette cavité, une fois dégagée, permettra d'accueillir le collier 110.

A l'aide d'une pointe carrée, le chirurgien réalise ensuite un trou de passage pour la vis 10, depuis la crête de l'os iliaque 201 jusque dans le corps de la vertèbre S1 du sacrum 202, en vérifiant que ce trou de passage traverse la cavité préalablement dégagée.

Le chirurgien retire ensuite cette pointe, place le collier 110 de l'élément de connexion 100 dans la cavité, puis engage une broche de guidage dans le trou de passage en prenant soin de vérifier que celle-ci traverse le trou axial 111 du collier 110.

La vis 10 est ensuite engagée sur cette broche de guidage et vissée solidement dans le bassin du patient, au travers de l'os iliaque 201, du trou axial 111 du collier 110, et du sacrum 202.

Une fois la vis 10 parfaitement ancrée dans le bassin du patient, le chirurgien replace si nécessaire le collier 110 sur la vis 10, puis il visse l'arrêtoir 150 dans le trou transversal 112 du collier 110 de manière à bloquer fixement le collier 110 sur la vis 10.

Le chirurgien place alors le réceptacle 130 dans la position désirée, en profitant des degrés de liberté offerts par la liaison rotule. Une fois cette position atteinte, le chirurgien engage la tige de liaison 3 dans le logement d'accueil 131 du réceptacle 130. Il visse ensuite le verrou 190 dans l'ouverture d'introduction 137 taraudée de manière à bloquer rigidement la tige de liaison 3 contre la bague 170 et contre les fonds des encoches latérales 135 du réceptacle 130, ce qui permet de bloquer la liaison rotule.

La tige de liaison 3 est ainsi parfaitement bloquée sur le bassin du patient.

La présente invention n'est nullement limitée au mode de réalisation décrit et représenté, mais l'homme du métier saura y apporter toute variante conforme à son esprit.

On pourrait notamment prévoir de remplacer le filetage du corps de l'arrêtoir par des dents d'encliquetage adaptées à s'accrocher à des griffes prévues en correspondance dans le trou transversal du collier. Dans cette variante, l'implant ilio-sacré serait alors livré avec l'arrêtoir positionné dans une position arrière, dans laquelle il ne bloquerait pas le passage de la vis dans le collier. Au cours de l'opération, cet arrêtoir serait alors adapté à être enfoncé par le chirurgien jusqu'à une position encliquetée sur les griffes du collier dans laquelle il bloquerait la vis dans le collier.

Selon une autre variante, on pourrait prévoir un implant ilio-sacré dont l'élément de connexion serait dépourvu de liaison rotule, de sorte que le collier et le réceptacle forment une seule pièce monobloc, ou forment deux pièces distinctes connectées l'une l'autre par une simple liaison pivot d'axe A2.

## Revendications

1. Implant ilio-sacré (2) pour système d'ostéosynthèse rachidienne (1), qui comprend un élément de connexion (100) comportant :
- un collier (110) qui présente un trou axial (111) traversant qui est destiné à être traversé par un élément d'ancrage (10), et un trou transversal (112) traversant qui débouche dans le trou axial (111),
- un réceptacle (130) qui est solidarisé au collier (110) et qui présente un logement d'accueil (131) d'une tige de liaison (3) du système d'ostéosynthèse rachidienne (1), et
- un arrêtoir (150) qui est destiné à bloquer l'élément d'ancrage (10) dans le trou axial (111) du collier (110),
**caractérisé en ce que** l'arrêtoir (150) comporte :
- un corps (151) qui est monté mobile au travers du trou transversal (112) du collier (110), et
- une partie de manoeuvre (153) qui est accessible par le logement d'accueil (131) du réceptacle (130) et qui permet de déplacer le corps (151) depuis une première position dans laquelle il laisse l'élément d'ancrage (10) libre de se mouvoir au travers du trou axial (111) du collier (110) jusqu'à une seconde position dans laquelle il bloque l'élément d'ancrage (10) dans le trou axial (111) du collier (110).

2. Implant ilio-sacré (2) selon la revendication précédente, dans lequel le trou transversal (112) du collier (110) est au moins en partie taraudé et dans lequel ledit corps comporte une tige filetée (151) vissée dans le trou transversal (112) du collier (110).

3. Implant ilio-sacré (2) selon la revendication précédente, dans lequel la partie de manoeuvre comporte une empreinte (153) en creux, dans laquelle un outil de vissage est adapté à être engagé pour visser la tige filetée (151) dans le trou transversal (112) du collier (110).

4. Implant ilio-sacré (2) selon l'une des revendications précédentes, dans lequel le logement d'accueil (131) du réceptacle (130) est délimité du côté du collier (110) par un fond (132) qui présente une ouverture (133) dans laquelle est engagé l'arrêtoir (150).

5. Implant ilio-sacré (2) selon l'une des revendications précédentes, dans lequel le réceptacle (130) est solidarisé au collier (110) au moyen d'une liaison rotule.

6. Implant ilio-sacré (2) selon la revendication précédente, dans lequel l'arrêtoir (150) comporte une tête (152) au moins partiellement sphérique ou conique, formant la partie mâle de ladite liaison rotule.

7. Implant ilio-sacré (2) selon les revendications 4 et 6, dans lequel le fond (132) du réceptacle (130) présente, autour de ladite ouverture (133), une face intérieure (134) sphérique ou conique sur laquelle s'appuie la tête (152) de l'arrêtoir (150) et qui forme la partie femelle de ladite liaison rotule.

8. Implant ilio-sacré (2) selon l'une des revendications 5 à 7, dans lequel l'élément de connexion (100) comporte un moyen de retenue (170) monté mobile dans le logement d'accueil (131) du réceptacle (130), entre une position de libération dans laquelle la liaison rotule est libre et une position de retenue dans laquelle la liaison rotule est bloquée pour rendre le collier (110) et le réceptacle (130) fixes l'un par rapport à l'autre.

9. Implant ilio-sacré (2) selon les revendications 7 et 8, dans lequel, le logement d'accueil (131) du réceptacle (130) débouchant vers l'extérieur par deux ouvertures latérales (135) en forme de berceaux et situées en regard l'une de l'autre, ledit élément de retenue comporte une bague (170) dont une face (171) s'appuie, en position de retenue, sur la tête (152) de l'arrêtoir (150), et dont une partie (172) s'étend, en position de libération, au-dessus des fonds des berceaux formés par lesdites deux ouvertures latérales (135).

10. Implant ilio-sacré (2) selon la revendication précédente, dans lequel ladite bague (170) est fendue et est engagée dans une gorge périphérique (136) située en creux dans le réceptacle (130), à l'intérieur du logement d'accueil (131).

11. Implant ilio-sacré (2) selon l'une des deux revendications précédentes, dans lequel ledit logement d'accueil (131) débouche vers l'extérieur à l'opposé du fond (132) par une ouverture d'introduction (137) taraudée, et dans lequel ledit élément de connexion (100) comporte un verrou (190) qui est vissé dans ladite ouverture d'introduction (137) pour immobiliser ladite tige de liaison (3) contre la bague (170) et contre les fonds des berceaux formés par lesdites deux ouvertures latérales (135).

12. Implant ilio-sacré (2) selon l'une des revendications précédentes, dans lequel il est prévu un élément d'ancrage (10) comportant une tige allongée (11) qui est adaptée à se fixer à l'os iliaque (201) d'un patient et qui est engagée dans le trou axial (111) du collier (110).

## Patentansprüche

1. Iliosakralimplantat (2) für ein spinales Osteosynthesesystem (1) mit einem Verbindungselement (100), das umfasst:
- einen Haltering (110) mit einem durchgehenden axialen Loch (111), um von einem Verankerungselement (10) durchquert zu werden, und ein durchgehendes transversales Loch (112), das in das axiale Loch (111) mündet,
- ein mit dem Haltering (110) formschlüssig verbundenes Receptaculum (130), das eine Aufnahme (131) für einen Verbindungsstift (3) des spinalen Osteosynthesesystems (1) aufweist, und
- eine Arretierung (150) zum Blockieren des Verankerungselements (10) im axialen Loch (111) des Halterings (110),
**dadurch gekennzeichnet, dass** die Arretierung (150) umfasst:
- einen durch das transversale Loch (112) des Halterings (110) beweglich gelagerten Körper (151), und
- einen über die Aufnahme (131) des Receptaculums (130) zugänglichen Betätigungsabschnitt (153), durch den der Körper (151) aus einer ersten Position, in der er das Verankerungselement (10) durch das axiale Loch (111) des Halterings (110) frei beweglich lässt, in eine zweite Position, in der er das Verankerungselement (10) im axialen Loch (111) des Halterings (110) blockiert, bewegt werden kann.

2. Iliosakralimplantat (2) nach vorausgehendem Anspruch, bei dem das transversale Loch (112) des Halterings (110) zumindest in einem Abschnitt ein Gewinde aufweist und bei dem der Körper einen in das transversale Loch (112) des Halterings (110) eingeschraubten Gewindestift (151) umfasst.

3. Iliosakralimplantat (2) nach vorausgehendem Anspruch, bei dem der Betätigungsabschnitt eine kreuzförmige Vertiefung (153) umfasst, in der ein Schraubwerkzeug angesetzt werden kann, um den Gewindestift (151) in das transversale Loch (112) des Halterings (110) einzuschrauben.

4. Iliosakralimplantat (2) nach einem der vorausgehenden Ansprüche, bei dem die Aufnahme (131) des Receptaculums (130) auf der Seite des Halterings (110) durch einen Boden (132) abgegrenzt ist, welcher eine Öffnung (133) aufweist, in die die Arretierung (150) gesteckt wird.

5. Iliosakralimplantat (2) nach einem der vorausgehenden Ansprüche, bei dem das Receptaculum (130) mittels einer Gelenkverbindung mit dem Haltering (110) formschlüssig verbunden ist.

6. Iliosakralimplantat (2) nach vorausgehendem Anspruch, bei dem die Arretierung (150) einen zumindest teilweise sphärischen oder kegelförmigen Kopf (152) umfasst, der das männliche Steckteil dieser Gelenkverbindung bildet.

7. Iliosakralimplantat (2) nach den Ansprüchen 4 und 6, bei dem der Boden (132) des Receptaculums (130) um die Öffnung (133) herum eine sphärische oder kegelförmige Innenseite (134) aufweist, auf der der Kopf (152) der Arretierung (150) aufliegt und die den weiblichen Aufnahmeteil dieser Gelenkverbindung bildet.

8. Iliosakralimplantat (2) nach einem der Ansprüche 5 bis 7, bei dem das Verbindungselement (100) ein beweglich in der Aufnahme (131) des Receptaculums (130) gelagertes Rückhaltemittel (170) umfasst, das sich zwischen einer frei beweglichen Position, in der sich die Gelenkverbindung frei bewegen kann, und einer Rückhalteposition, in der die Gelenkverbindung blockiert ist, um den Haltering (110) und das Receptaculum (130) gegenseitig fest zu halten, bewegen kann.

9. Iliosakralimplantat (2) nach den Ansprüchen 7 und 8, bei dem, während die Aufnahme (131) des Receptaculums (130) über zwei seitliche, wiegenförmige und gegenüberliegende Öffnungen (135) nach außen mündet, das Rückhalteelement einen Ring (170) umfasst, dessen eine Seite (171) in der Rückhalteposition auf dem Kopf (152) der Arretierung (150) aufliegt, und von dem sich ein Abschnitt (172) in der frei beweglichen Position über den durch die beiden seitlichen Öffnungen (135) gebildeten Wiegenböden erstreckt.

10. Iliosakralimplantat (2) nach vorausgehendem Anspruch, bei dem der Ring (170) gespalten und in eine periphere Nut (136) eingesetzt ist, die sich auf der Innenseite der Aufnahme (131) im Receptaculum (130) vertieft befindet.

11. Iliosakralimplantat (2) nach einem der zwei vorausgehenden Ansprüche, bei dem die Aufnahme (131) entgegengesetzt zum Boden (132) über eine Einführöffnung (137) mit Gewindebohrung nach außen mündet, und bei dem das Verbindungselement (100) einen Riegel (190) umfasst, der in die Einführöffnung (137) geschraubt wird, um den Verbindungsstift (3) am Ring (170) und an den durch die zwei seitlichen Öffnungen (135) gebildeten Wiegenböden zu blockieren.

12. Iliosakralimplantat (2) nach einem der vorausgehenden Ansprüche, bei dem ein Verankerungselement (10) mit einem länglichen Stift (11) vorgesehen ist, der am Hüftknochen (201) eines Patienten fixiert werden kann und in das axiale Loch (111) des Halterings (110) gesteckt wird.

## Claims

1. An iliosacral implant (2) for a spinal osteosynthesis system (1) that includes a connection element (100) comprising:
• a collar (110) having an axial through hole (111) for having an anchor element (10) passing therethrough, and a transverse through hole (112) that opens out into the axial hole (111);
• a receptacle (130) that is connected to the collar (110) and that presents a reception housing (131) for receiving a connection rod (3) of the spinal osteosynthesis system (1); and
• a stopper (150) for blocking the anchor element (10) in the axial hole (111) of the collar (110);
the implant being **characterized in that** the stopper (150) comprises:
• a body (151) that is movably mounted through the through hole (112) of the collar (110); and
• a drive portion (153) that is accessible via the reception housing (131) of the receptacle (130) and that enables the body (151) to be moved from a first position in which it leaves the anchor element (10) free to move through the axial hole (111) of the collar (110) to a second position in which it blocks the anchor element (10) in the axial hole (111) of the collar (110).

2. An iliosacral implant (2) according to the preceding claim, wherein the through hole (112) of the collar (110) is tapped at least in part and wherein said body includes a threaded rod (151) screwed into the through hole (112) of the collar (110).

3. An iliosacral implant (2) according to the preceding claim, wherein the drive portion includes a socket (153) into which a driver tool is adapted to be engaged in order to screw the threaded rod (151) into the through hole (112) of the collar (110).

4. An iliosacral implant (2) according to any preceding claim, wherein the reception housing (131) of the receptacle (130) is defined beside the collar (110) by an end wall (132) that presents an opening (133) in which the stopper (150) is engaged.

5. An iliosacral implant (2) according to any preceding claim, wherein the receptacle (130) is connected to the collar (110) by means of a ball-joint connection.

6. An iliosacral implant (2) according to the preceding claim, wherein the stopper (150) includes a head (152) that is at least partially spherical or conical, forming the male portion of said ball-joint connection.

7. An iliosacral implant (2) according to claims 4 and 6, wherein the end wall (132) of the receptacle (130) presents a spherical or conical inside face (134) around said opening (133), against which the head (152) of the stopper (150) comes to bear, and forming the female portion of said ball-joint connection.

8. An iliosacral implant (2) according to any one of claims 5 to 7, wherein the connection element (100) includes retaining means (170) movably mounted in the reception housing (131) of the receptacle (130) to move between a release position in which the ball-joint connection is free, and a retaining position in which the ball-joint connection is blocked to cause the collar (110) and the receptacle (130) to be stationary relative to each other.

9. An iliosacral implant (2) according to claims 7 and 8, wherein the reception housing (131) of the receptacle (130) opens to the outside via two lateral openings (135) in the form of cradles and situated facing each other, and said retaining element includes a ring (170) having a face (171) that, in the retaining position, bears against the head (152) of the stopper (150), and with a portion (172) that, in the release position, extends over the backs of the cradles formed by said two lateral openings (135).

10. An iliosacral implant (2) according to the preceding claim, wherein said ring (170) is split and is engaged in a peripheral groove (136) set back in the receptacle (130) inside the reception housing (131).

11. An iliosacral implant (2) according to either one of the two preceding claims, wherein said reception housing (131) opens to the outside away from the end wall (132) via a tapped insertion opening (137), and wherein said connection element (100) includes a nut (190) that is screwed into said insertion opening (137) to hold said connection rod (3) stationary against the ring (170) and against the end walls of the cradles formed by said two lateral openings (135).

12. An iliosacral implant (2) according to any preceding claim, wherein an anchor element (10) is provided that includes an elongate rod (11) that is adapted to be fastened to the iliac bone (201) of a patient and that is engaged in the axial hole (111) of the collar (110).
